# EUROPEAN PATENT APPLICATION

(11) **EP 2 703 003 A1**
(43) Date of publication of application: **05.03.2014**
(21) Application number: 12380043.5
(22) Date of filing: 31.08.2012
(51) Int. Cl.: A61K 36/185, A61P 19/00, A61P 17/14

(54) **A pomegranate extract and compositions containing pomegranate polyphenols for treating or preventing diseases or physiopathological conditions associated with an excessive gene expression and/or physiological activity of interleukin-6**

(71) Applicant: Probelte Biotecnologia S.L., 30100 Espinardo Murcia (ES)
(72) Inventor: Streitenberger, Sergio A., 30120 El Palmar (Murcia) (ES); Lopez Mas, José A., 30840 Alhama de Murcia (Murcia) (ES)
(74) Representative: Gislon, Gabriele

(57) **Abstract**

The invention describes compositions containing pomegranate extract containing polyphenols as an active ingredient and combinations of said extract with other nutrients or drugs for use in preventing, treating or co-treating diseases or physiopathological conditions associated with excessive interleukin-6 gene expression and/or excessive activation of the physiological mechanisms mediated interleukin-6 actuation among which those presenting bone mass loss, progressive cartilage loss and hair loss should be highlighted.

## Description

### Field of the Invention

The present invention relates to a pomegranate extract and to compositions containing pomegranate extract containing polyphenols as an active ingredient and various combinations of said extract with other nutrients for use in preventing, treating or co-treating diseases or physiopathological conditions associated with excessive interleukin-6 gene expression and/or excessive activation of the physiological mechanisms mediated by interleukin-6 actuation among which those presenting with bone mass loss, progressive cartilage loss and hair loss should be highlighted.

Methods for promoting a balance between bone formation and resorption, reducing progressive cartilage loss, increasing hair growth, reducing hair loss, increasing hair follicle viability, or increasing hair follicle cell viability are also described, said methods comprising the administration to a mammal of a composition including pomegranate extract as an active ingredient, having the capacity to down-regulate interleukin-6 gene expression, wherein the pomegranate extract is in the form of a composition that can be administered by oral route, or a composition that can be administered by topical route, or in the form of a mixed set comprising the composition that can be administered by oral route and the composition that can be administered by topical route.

### Background of the Invention

Interleukin-6 is a multifunctional protein involved in several effects, including B cell differentiation into plasma cells, T cell activation, stimulation of fever, or release of acute-phase proteins by hepatocytes and activation of the complement cascade.

Excessive interleukin-6 gene expression and/or excessive activation of the physiological mechanisms mediated interleukin-6 actuation in different tissues act as mediators of different physiological effects that can cause diseases or physiopathological conditions

A number of research work suggest that interleukin-6 plays a key role in the process of activating immature osteoclasts, which are the bone cells responsible for bone resorption. In addition, recent studies indicate that estrogens inhibit production of interleukin-6 by osteoblasts (bone cells responsible for new bone formation) and bone marrow stromal cells. The balance between bone resorption by osteoclasts and the bone formation by osteoblasts regulates bone metabolism. The drop in the estrogen production in women during post-menopause with high prevalence leads to an accelerated bone mass loss, which clearly correlates with the loss of estrogen production and can be prevented by means of early estrogen replacement therapy. If accelerated bone mass loss is not slowed down by means of suitable treatment, it can result in reduced bone strength predisposing a person to an increased risk of fracture.

Hormone replacement therapy replaces the hormone deprivation due to the cease of ovarian activity. There is efficacy data about bone mineral density (BMD) from randomized controlled trials RCT. Treatment induces a slight but significant increase in the risk of detecting breast cancer, more evident in prolonged treatments, (Level of evidence 2a) and of thromboembolic disease (Level of evidence 1b).

A number of plants and compounds derived from plants have been used in preventing bone mass loss. Remarkable examples are soy isoflavones (e.g. genistein) or resveratrol, in both being substances having a phytoestrogen nature. Resveratrol is a potent phytoestrogen found in grape peels and in other plant foods, as well as in wine. Studies have demonstrated that resveratrol forms a complex with estrogen receptors, and it can help women maintain normal estrogen activity and maintain healthy bone density.

The phytoestrogen effect of soy isoflavones is also well-known, and results suggest that osteoblast function is promoted by soy extract and that the estrogen receptor is involved in the response, it therefore plays an important role in bone remodeling.

It should be pointed out that the compounds derived from plants that have been used in preventing bone mass loss, exemplified by soy isoflavones and resveratrol, exert their effect through estrogen receptors.

Other research work indicates that increased interleukin-6 levels make this protein an important therapeutic target in the diseases presenting progressive cartilage loss due to its capacity to stimulate differentiation of the osteoclast that may be responsible for joint destruction. Tocilizumab (TCZ), a new biological drug acting as an antibody against interleukin-6 receptors blocking its binding, has recently been added to the therapeutic arsenal. TCZ is the first biological treatment acting against this therapeutic target. It is an inhibitory monoclonal antibody of interleukin-6 receptor.

Interleukin-6 is stimulated by viral and bacterial components and is one of the most important innate response mediators. The most common side effects described in studies controlled with TCZ were respiratory tract infections, nasopharyngitis, headache, hypertension and increased transaminases. Serious infections and the hypertransaminemia were the two most common causes for interrupting treatment. The most common cause of death in patients undergoing treatment with TCZ was sepsis.

Hair loss is a physiological phenomenon, and it is considered pathological only when it is excessive or when the pattern is abnormal. The physiological cycle of hair consists of 3 phases:
- Anagen or growth phase: 90% of hair is in this phase, with a growth of 0.3 mm a day.
- Catagen or resting phase: this is a transitional phase in which hair ceases to grow. Approximately 1% of hair is in this phase.
- Telogen or loss phase: this phase lasts about 3 months. Hair falls out and a new hair follicle appears during this phase.

Alopecia is defined as hair loss of any type or origin. Alopecia is classified according to its clinical morphology as scarring and non-scarring alopecia. Scarring alopecia occurs as the result of the malformation, damage or destruction of pilosebaceous follicles, which are replaced with scar cicatricial such that it can no longer produce hair.

Non-cicatricial alopecia forms a more complex group and can be due to two fundamental situations, i.e., the entrance of many follicles in the telogen phase or the transformation of terminal hair into vellus hair.

Androgenetic alopecia (AGA) or pattern baldness consists of progressive hair loss induced by the action of androgens. It affects both men and women but follows a different pattern of evolution, known as Hamilton pattern baldness in men and as Ludwig pattern baldness in women.

It is a normal process in men in which two factors are involved, genetic predisposition and the existence of suitable androgen levels. Alopecia is only a problem when it is premature or is not accepted by the patient.

AGA is the result of increased production of 5-dihydrotestosterone hormone, which is a metabolite of the testosterone produced by the action of 5-α-reductase and induces hair miniaturization. Androgen receptors and enzymes metabolizing androgens (5-α-reductase and aromatase CP-450) are involved in the action of androgens at the scalp level.

The different clinical patterns of presentation of AGA in men and women are determined by the different concentration of androgen receptors (40% less in women than in men) and by the different levels of 5-α-reductase (higher number in men and in the frontal region) and of aromatase CP-450 (60% higher in women).

Another non-cicatricial alopecia is alopecia areata; it is a common form of alopecia affecting 1-2% of the population in which a high percentage of hairs of the affected area enter the telogen phase. It can be developed in any area of the body covered in hair

Treatment can include the administration of corticoids, topical anthralins, minoxidil and, in severe cases or in cases with little response to treatments, the establishment of topical immunotherapy by means of using sensitizing agents such as diphencyprone the purpose of which is to reduce the peribulbar inflammatory component.

Other non-cicatricial alopecias are associated with systemic diseases.

In recent years, pharmacological therapies have proven to be the most effective treatments for controlling non-cicatricial alopecia, mainly androgenetic alopecia. The two most effective drugs are minoxidil and finasteride.

Minoxidil is described in patent US 4596812. Its mechanism of action is unclear, although there are *in vitro* studies which point to acting directly on hair follicle cells (being capable of inducing the production of growth factors increasing vascularization in that area. The main problem of this product is that it can cause various side effects such as itchy scalp (1.5% of patients) and less frequently irritative dermatitis, non-specific allergic reactions, urticaria, allergic rhinitis, facial swelling, sensitivity, dyspnea, headaches, neuritis, confusion, etc.... Other reactions such as contact dermatitis, folliculitis, alopecia, unwanted hypertrichosis and seborrhea have rarely been detected.

Finasteride is a 5-α-reductase type II inhibitor, an enzyme converting testosterone into 5-dihydrotestosterone (DHT), and is used as systemic therapy in men, according to European patent EP 0724444. It must be administered for long time periods (at least 1 year) at a dose of 1 mg/day for it to be effective. Efficacy of this drug has been tested in various studies. Adverse effects such as erectile dysfunction, ejaculation disorders, reduced libido or gynecomastia have also been described. This drug is contraindicated in women due to the risk it involves in pregnancy because its capacity to inhibit 5-α-reductase can cause external genital anomalies in masculine fetuses.

Both in the case of minoxidil and in the case of finasteride, treatment must be administered for long time periods and their effect disappears when treatment is ended.

It has been demonstrated that vitamin deficiency can be one of the causes of non-cicatricial alopecia, so in this case the logical treatment would be to take vitamins.

There are supplements and preparations containing biotin on the market that are advertised as "tested" solutions in preventing hair loss or repairing damaged hair. Other vitamin supplements claiming to be useful in preventing hair loss or enhancing hair growth are also sold, although in no case have they shown to have any relationship with alopecia or to be effective in the absence of a vitamin deficiency. Together with these treatments, there are many other substances forming part of hair lotions and creams such as zinc, amino acids, various vitamins, hormones, jojoba oil, urea... which, when administered as coadjuvant treatments, do not normally have side effects, are less invasive than transplants and surgery and have palliative effects in alopecia, usually through moderate hair loss prevention.

Application US20060120980 (A1) describes dermatological compositions acting on follicle cells and methods related to hair growth, dermal repair and enhancing skin health.

Application US20070036742 describes a method for modulating hair growth or regrowth comprising administering a composition comprising one or more ingredients among which several extracts are mentioned, wherein the composition has one or more of the following functions: inhibition of dihydrotestosterone (DHT) synthesis, inhibition of proteasome activity, inhibiting IL-1 activity, increased secretion of keratinocyte growth factor (KGF), or increased secretion of the vascular endothelial growth factor (VEGF).

Application US20100247428 describes the use of compositions containing as an active ingredient chemical elements belonging to the group of rare earth elements (e.g. cerium, europium, etc...) for preventing hair loss and promoting hair growth based on their capacity to down-regulate TGF-B2 expression (preventing hair follicle cell apoptosis) and promoting VEGF expression to enhance blood flow through vasodilating action, which improves the supply of nutrients to hair root cells and promotes hair growth.

In summary, the state of the art described above includes different inventions which have described active ingredients for preventing, treating or co-treating diseases or physiopathological conditions presenting bone mass loss, progressive cartilage loss and hair loss, but which have various side effects and require a large number of applications and care related thereto, so the search for new active ingredients and methods with fewer side effects which require fewer and more effective applications than those currently known in the state of the art must continue.

The object of the present invention is to solve the aforementioned problems by means of using an extract, compositions and active ingredients having the capacity to be used to down-regulate interleukin-6 gene expression, for promoting a balance between bone formation and resorption, reducing progressive cartilage loss, favoring hair growth and stopping hair loss by means of the oral and/or topical administration in a mammal including its application in humans for improving human hair health, promoting a balance between bone formation and resorption and reducing progressive cartilage loss.

### Description of the Invention

The bioactive compounds of pomegranate (e.g. polyphenols and particularly punicalagins which are very soluble in water) have demonstrated potential health benefits in various aspects: prevention of cardiovascular diseases, prevention of degenerative diseases, decreased risk of carcinogenic diseases, anti-inflammatory properties, antiparasitic, antiviral and antimicrobial activity, treatment of individuals with low sperm count and/or quality and treatment of erectile dysfunction.

The present inventors have surprisingly found that plant extracts standardized in terms of the content of these same compounds, polyphenols and particularly punicalagins, have the capacity to promote a balance between bone formation and resorption, reducing progressive cartilage loss, stopping hair loss and promoting hair growth in humans, and that remarkably these effects are not achieved through the ways described in the prior state of the art anterior relating to:
- effect of plant phytoestrogens and their binding to estrogen receptors in hormone deprivation conditions due to the cease of ovarian activity (e.g. in post-menopause),
- inhibition of dihydrotestosterone (DHT) synthesis through 5-α-reductase type II enzyme inhibition,
- increased KGF secretion,
- increased VEGF secretion,
- down-regulating TGF-B2 expression, etc....

In contrast, multiple pieces of evidence found by the inventors of the present invention indicate that pomegranate extract standardized to polyphenols and/or punicalagins has the capacity to promote a balance between bone formation and resorption, reducing progressive cartilage loss, stopping hair loss and promoting hair growth in humans.

Evidence indicates that this effect is produced by the capacity of pomegranate polyphenols in general and of punicalagins in particular to down-regulate interleukin-6 gene expression in diseases or physiopathological conditions associated with excessive interleukin-6 gene expression and/or excessive activation of the physiological mechanisms mediated by interleukin-6 actuation, which had not been disclosed until now.

The present invention additionally shows evidence that treatment with pomegranate extract containing polyphenols as an active ingredient inhibits THBS1 gene expression, activates LPAR6 gene expression and both gene regulation effects relate to stopping hair loss and promoting hair growth in humans.

The authors of the present invention have additionally found that oral administration in mice with collagen type II-induced arthritis of a pomegranate extract containing pomegranate polyphenols as an active ingredient inhibits the CD36 gene expression in the cartilage tissue. It is known through existing scientific publications that CD36 gene expresses a protein known as thrombospondin receptor, and it has been found that in cartilage with severe osteoarthritis there is a significant increase in the number of chondrocytes stained by immunostaining due to the presence of CD36 protein on the surface. Furthermore, in cartilage with moderate osteoarthritis, CD36 protein expression (shown by RNA measurements) increased significantly, predominantly in superior cartilage.

In another published study where differences in gene transcription profiles in osteoporotic bone samples and non-osteoporotic bone samples from 2 groups of post-menopausal women were identified for the first time by means of real time PCR, there was found to be a significant increase in CD36 gene expression in osteoporotic bone samples. Specifically, it was found that the change in relative gene expression in women with osteoporosis compared to the non-osteoporotic controls had a value of 3.22, this increase in gene expression being statistically significant (p = 0.03).

Through earlier publications relating CD36 overexpression in osteoarthritic cartilage and osteoporotic bone and through the inventors' finding that treatment with pomegranate extract containing polyphenols as an active ingredient inhibits CD36 gene expression, it is proven that the effect of treatment with pomegranate extract containing polyphenols as an active ingredient on gene regulation is related to stopping bone mass loss and progressive cartilage loss.

The present invention claims pomegranate extract containing polyphenols as an active ingredient for use in treating, preventing or co-treating diseases or physiopathological conditions associated with excessive interleukin-6 gene expression and/or excessive activation of the physiological mechanisms mediated interleukin-6 actuation in mammals. The pomegranate extract containing polyphenols of the invention is particularly useful for treating those conditions presenting bone mass loss, progressive cartilage loss and hair loss.

It must be stated that when reference is made to diseases or physiopathological conditions associated with excessive interleukin-6 gene expression and/or excessive activation of the physiological mechanisms mediated interleukin-6 actuation in the context of the present invention, diseases or physiopathological conditions such as hair loss, alopecia, and others such as necrosis, inflammation, edema, myocardial infarction, headaches, hypotension, hyperalgesia, heart failure, fever, atherosclerosis, convulsions, myocardial ischemia, breast neoplasm, hepatocellular carcinoma, cirrhosis of the liver, depressive disorders, cerebrovascular accidents, ventricular dysfunction, multiple myeloma, brain damage, reperfusion injury, ischemia, glomerulonephritis, ovarian neoplasm, hemolytic uremic syndrome, systemic lupus erythematosus, pulmonary fibrosis, diabetes mellitus type 2, hypercalcemia, neurodegenerative diseases, metabolic syndrome, osteoporosis, post-menopausal osteoporosis, arthritis periodontal disease, respiratory hypersensitivity, obesity, aseptic meningitis, polyomyositis, Kaposi's sarcoma, cachexia, cholangiocarcinoma, atopic dermatitis, periodontal disease, psoriasis, inflammatory bowel disease, autism, schizophrenia, Crohn's disease, burns, secondary hyperparathyroidism, autoimmune hepatitis, mitochondrial myopathies, entamoebiasis, leishmaniasis, diaphragmatic hernia, HIV wasting syndrome, berylliosis, prostate tumors, drug-induced liver damage, acute kidney injury, peripheral nervous system diseases or amnesia, are understood to be comprised in this meaning.

By means of the present invention, it has been demonstrated that a pomegranate extract rich in polyphenols, preferably one rich in punicalagins which are very soluble in water and have high *in vivo* bioavailability due to their low free ellagic acid content that is very slightly soluble in water and scarcely bioavailable (with a punicalagin/free ellagic acid % by weight ratio in the range of 2/1 to 40/1), such as the extracts obtained according to EP 1 967 079 A1 (belonging to PROBELTE PHARMA and included herein by reference) respectively, act as active ingredients, having the capacity to down-regulate interleukin-6 gene expression, promoting a balance between bone formation and resorption, reducing progressive cartilage loss, promoting hair growth and stopping hair loss.

The present invention claims compositions containing pomegranate extract containing polyphenols as an active ingredient for use in promoting a balance between bone formation and resorption, reducing progressive cartilage loss, favoring hair growth and stopping hair loss by means of the oral and/or topical administration in a mammal including their application in humans to improve bone, joint cartilage and hair health.

The present specification also describes methods for promoting a balance between bone formation and resorption, reducing progressive cartilage loss, increasing hair growth, reducing hair loss, increasing hair follicle viability, or increasing hair follicle cell viability, comprising the administration in said mammal of a composition including pomegranate extract containing polyphenols as an active ingredient, having the capacity to down-regulate interleukin-6 gene expression, in which the pomegranate extract is in the form of a composition that can be administered by oral route, or of a composition that can be administered by topical route or in the form of a mixed set comprising the composition that can be administered by oral route and the composition that can be administered by topical route.

In one aspect of the invention, the methods for increasing hair growth, reducing hair loss, increasing hair follicle viability, or increasing hair follicle cell viability, comprising the administration in said mammal of a composition including pomegranate extract containing polyphenols as an active ingredient, having the capacity to down-regulate interleukin-6 gene expression, in combination with drugs such as minoxidil or finasteride, the doses of minoxidil or finasteride used in combination with pomegranate extract being significantly lower than those used in standard treatments with minoxidil or finasteride. Therefore, by means of the synergistic actuation of pomegranate extract with these drugs, their side effects are reduced.

In a particular case, the method of capillary health treatment comprises the oral administration of a composition containing finasteride at a dose of 0.5 mg/day (50% lower than the standard dose) or less, and polyphenols of a pomegranate extract at a dose between 10 and 1500 mg/day, preferably at least 50 mg/day, and more preferably at least 100 mg/day (expressed as gallic acid equivalent) capable of down-regulating interleukin-6 gene expression.

The inventors of the present invention found in *in vitro* assays that interleukin-6 inhibited elongation of the hair in a dose-dependent manner, and that in vitro treatment of the dermal papilla hair follicle cells with DHT also caused the release of interleukin-6. These findings strongly suggest that DHT binding to its receptors in dermal papilla hair follicle cells causes the release of interleukin-6 inhibiting the hair elongation. Accordingly, synergy is achieved between the inhibition in the production of DHT by finasteride and down-regulation in the production of interleukin-6, a dose-dependent effect with administrations of pomegranate extract polyphenols

The side effects of finasteride such as erectile dysfunction, ejaculation disorders, decreased libido or gynecomastia are thereby reduced by the lower dose of the finasteride drug used but also by the synergy with pomegranate extract the bioactive compounds of which (e.g. polyphenols and particularly punicalagins) have demonstrated potential benefits in the treatment of individuals with a low sperm count and/or quality and treatment of erectile dysfunction.

In another particular case, the method of capillary health treatment comprises the topical administration of a composition containing minoxidil, which acts directly on hair follicle cells (being capable of inducing the production of growth factors which increase vascularization in that area), in the form of a topical solution at a concentration of 1 % (50% less than the lowest standard concentration), and pomegranate extract in the form of topical solution, the dose being between 1 and 16 ml/day, at a pomegranate polyphenol concentration of 0.2% to 3.2% capable, as found by the present inventors, of down-regulating interleukin-6 and thrombospondin 1 gene expression (see details in Example 6). Thrombospondin 1 plays a critical role in inducing follicle regression and the follicle passing on to the catagen phase through its inhibitory effect on angiogenesis. Accordingly, synergy is achieved between increased vascularization (angiogenesis) in the root area of the hair follicle due to minoxidil and down-regulation in the production of thrombospondin 1 (which entails protection against the thrombospondin 1-mediated angiogenesis regression effect), a dose-dependent effect with administrations of pomegranate extract polyphenols.

The side effects of minoxidil, such as itchy scalp, irritative dermatitis, non-specific allergic reactions, urticaria, allergic rhinitis, facial swelling, sensitivity, dyspnea, headaches, neuritis, confusion, etc..., are thereby reduced by the lower dose of the minoxidil drug used.

A pomegranate extract containing pomegranate polyphenols preferred for promoting a balance between bone formation and resorption, reducing progressive cartilage loss, increasing hair growth, reducing hair loss, increasing hair follicle viability, or increasing hair follicle cell viability is that containing punicalagins and free ellagic acid, in which the punicalagin content is at least 2% (w/w) and the free ellagic acid content is such that the punicalagin/free ellagic acid ratio (% w/w) is in the range of 2/1 to 40/1, and the total phenol content is additionally at least 5% (w/w) (expressed as gallic acid equivalent).

The composition of the pomegranate extract indicated above is particularly useful for using pomegranate polyphenols as active ingredients for promoting a balance between bone formation and resorption, reducing progressive cartilage loss, promoting hair growth and stopping hair loss due to its pomegranate polyphenol content and particularly to its punicalagin content, which are very soluble in water and have high *in vivo* bioavailability, and to their low free ellagic acid content which is very scarcely soluble in water and scarcely bioavailable, such that the punicalagin/free ellagic acid ratio (% w/w), is in the range of 2/1 to 40/1, preferably at least 14/1. Therefore, it is desirable for the pomegranate extracts developed for use as active ingredients for promoting a balance between bone formation and resorption, reducing progressive cartilage loss, promoting hair growth and stopping hair loss to have the highest possible punicalagin/free ellagic acid ratio (% w/w).

Another preferred pomegranate extract is that obtained according to application EP 1967079 and having a punicalagin content of at least 5% (w/w), preferably at least 20% (w/w) and more preferably at least 40% (w/w), and a free ellagic acid content such that the punicalagin/free ellagic acid ratio (% w/w) is in the range of 2/1 to 40/1, preferably at least 14/1, the total phenol content is at least 10% (w/w), preferably at least 50% (w/w) and more preferably at least 75% (w/w) (expressed as gallic acid equivalent), and water solubility is at least 1% (w/w), preferably at least 5% (w/w) and more preferably at least 7% (w/w) (measured according to ASTM E 1148-02). The extract preferably has a residual organic solvent content less than 1 ppb and more preferably it is 0 ppm).

The pomegranate extract the use of which is proposed preferably contains at least 50% (w/w) total polyphenols (expressed as gallic acid equivalent) and the amount of punicalagins (a type of active ingredient present in the pomegranate and included in the total polyphenols of pomegranate) being at least 20% (w/w) of the extract.

Another pomegranate extract preferred in the present invention for use as active ingredients for promoting a balance between bone formation and resorption, reducing progressive cartilage loss, promoting hair growth and stopping hair loss is that obtained according to application EP 1967079 characterized by being a pomegranate extract containing at least 1.5% (w/w) punicalins and having a punicalins/free ellagic acid ratio (% w/w) in the range of 2/1 to 40/1.

The substantial absence of even minimal traces of organic solvents such as methanol, ethanol, isopropanol, which are used in purification steps commonly used in the art, is very important for using the aforementioned extracts.

An additional advantage of the aforementioned extracts is the purification against the residues of plant health compounds present in the raw material, i.e., in pomegranates. Potentially toxic substances such as insecticides, herbicides, fungicides, rodenticides, etc., such as diuron, terbuthylazine, simazine, α-endosulfan, and β-endosulfan, can therefore be present in the starting material.

The aforementioned problem was solved in the preparation of pomegranate extracts rich in polyphenols and particularly in punicalagins obtained according to application EP 1 967 079 A1, given that non-ionic absorption resins were specifically selected so that it was possible to elute the active compounds of the mentioned extracts by means of using only water or a basified aqueous solution and not by means of using organic solvents.

It is additionally important to consider another important point. Several authors have found that certain toxic alkaloids (e.g. pelletierine, isopelletierine and pseudopelletierine) can be present when alcohols were used to obtain pomegranate extracts, however alkaloids are not detected when the pomegranate extracts are prepared using only water as occurs when the pomegranate extract rich in punicalagins is obtained according to application EP 1 967 079 A1.

The pomegranate extract rich in pomegranate polyphenols and particularly in punicalagins obtained according to application EP 1 967 079 A1 is alkaloid-free because no organic solvent is used in any of the extract production steps.

In another aspect of the present invention, compositions containing pomegranate extract containing pomegranate polyphenols as an active ingredient and various combinations of said extract with other nutrients (biotin, B2, B6, B12, vitamin A, vitamin C, vitamin D, folic acid, Fe, Cu, Se, I, Zn, L-methionine, L-cysteine, for-amino benzoic acid (PABA), linoleic acid, pantothenic acid, etc...) are claimed for the specified uses for favoring hair growth and stopping hair loss by means of the oral and/or topical administration in a mammal, including application in humans, for improving hair health.

The amount of pomegranate extract contained in the claimed compositions as an active ingredient can be comprised between 0.5% and 100% (w/w) of the composition to use for favoring hair growth and stopping hair loss by means of the oral and/or topical administration in a mammal, including application in humans, for improving hair health.

In another aspect of the present invention, compositions containing pomegranate extract containing pomegranate polyphenols as an active ingredient and various combinations of said extract with the active ingredients of drugs commonly used in capillary health, such as minoxidil or finasteride, are claimed for the specified uses. Pomegranate extracts containing pomegranate polyphenols can act at fairly low doses while at the same time their co-administration allows reducing the doses and concentrations of minoxidil or finasteride in the compositions that can be applied topically or orally based on combinations of pomegranate extracts with minoxidil or finasteride, and said compositions advantageously have fewer side effects than when treatment consists of the administration per se of the drug with minoxidil or finasteride.

The present invention will be described below in reference to the enclosed examples, tables and drawings in a non-limiting manner.

### Description of Tables and Drawings

Table 1 shows the scheme according to which the clinical pilot study on hair health described in Example 1 was performed.
Table 2 shows a summary of results discussed in Example 2 (pull test and phototrichogram) for the delivered treatment of the clinical pilot study on hair health.
Tables 3 and 4 show the results of the non-parametric test for related samples, using the Wilcoxon statistic, for the analyzed parameters, glucose and GPT, in individuals who received treatments 1 and 2 respectively, discussed in Example 4.
Tables 5 to 8 are related to the intergroup statistical analysis (of two in two groups) of the differences found between the day 0 and day 30 visit in different parameters of the phototrichogram (results discussed in Example 8).
Figures 1, 2 and 3 show the average amount of hairs in the telogen phase, anagen phase and the ratio of the average amount of hairs in the anagen/telogen phase, respectively, per visit in subjects who received treatments 1 or 2 (results discussed in Example 3).
Figure 4 shows the results described in Example 5 on differential gene expression of the interleukin-6 gene in an animal model of bovine collagen type II-induced cartilage inflammation and degradation, expressed as a value of the interleukin-6/GAPDH ratio.
Figure 5 shows the results described in Example 5 on the reduction of the severity of collagen type II-induced arthritis in mice.
Figure 6 shows the results described in Example 6 on the interleukin-6 serum level in a nutritional intervention study in humans.
Figure 7 shows the results described in Example 10 on total bone mineral density in a preclinical study in rats.

### Examples

### Example 1. Longitudinal, randomized, clinical pilot study for evaluating the healthy properties and tolerability of two treatments on preventing hair loss in a population with non-cicatricial alopecia.

The population sample consisted of 20 men.

Inclusion/exclusion criteria:
● Male subjects.
● Age between 18 and 65 years old, both included.
● Having non-cicatricial alopecia in stages II to IV according to the Hamilton scale.
● Having not received any treatment for hair loss during the 3 months prior to the beginning of the study.
● Not using hair dyes or treatments susceptible to damaging hair (perms...) within at least two weeks before the beginning of the study.
● Not being allergic to the components of the product under study.
● Suitable level of culture and comprehension of the clinical study.
● Agreeing to voluntarily participate in the study and giving a written informed consent.

The subjects were randomly included in one of the two intervention groups, and treatment was applied according to the sponsor's instructions.

Experimental treatments:
● Orally administered nutricosmetic treatment for preventing hair loss (hereinafter Treatment 1).
● Topically administered local treatment by means of hair lotion for preventing hair loss (hereinafter Treatment 2).

The following chart shows the quantitative composition in terms of the active ingredients of the product under study:

| | Experimental | Experimental | |
|---|---|---|---|
| PRODUCT | Food supplement | Hair lotion PB08 | |
| NAME | POMANOX | | |
| COMPOSITION (%): | Pomegranate extract^{$} 380 mg | 2.5% Pomegranate extract^{$} (w/V) | |
| | pomegranate extract/capsule | 1% D-Pantenol (w/V) | |
| | | 0.5% zinc PCA (w/V) | |
| | | 5% ethanol (w/V) | |
| | | 0.1 % isothiazoline (w/V) | |
| DOSE: | 2 capsules/day (280 mg polyphenols/day) | 4 ml/day (37 mg polyphenols/day) | |
| ADMINISTRATION ROUTE: | Oral | Topical | |
| DOSAGE FORM: | Capsules | Liquid | |
| MANUFACTURING LAB.: | Probelte Pharma Laboratories | Probelte Pharma Laboratories | |

| | | | |
|---|---|---|---|
| $: standardized in pomegranate polyphenols (37%) and in α + β punicalagins (15%): | | | |

The treatments were applied by the patient according to the sponsor's instructions, which are described below.

The food supplement (Treatment 1) was delivered in capsules, and the subject had to take two a day, one in the morning after breakfast and the other on after lunch or dinner.

The subjects had to apply the hair lotion (Treatment 2) daily, at night, in the affected area with 30 sprays using the sprayer (approximately 4 ml). It was left to act for two hours and was subsequently rinsed, if necessary.

The study had 2 phases during development:
A) Phase of selection:
   The participants were selected by means of anamnesis and physical examination (selection visit) between four and eight weeks prior to the beginning of the experimental phase.
B) Experimental phase:
   Each subject went through a 60-day experimental period. While the experimental phase lasted, the subject made 3 visits to the clinic (Days 0 - 30 - 60) to evaluate the parameters relating to the signs and symptoms of non-cicatricial alopecia such as hair density, regeneration, hair strength, shine and volume. A scheme of the study is shown in Table 1.

**Table 1**

| t follow-up | Study selection | Day 0 | Day 30 | Day 60 |
|---|---|---|---|---|
| Medical history | + | | | |
| Consent | + | | | |
| Inc/excl crit | + | + | | |
| Randomization | | + | | |
| Admin. of treatments | | + | + | + |
| Intervention | | +++++++++++++++++++++++++ | | |

| Control | VS | V1 | V2 | V3 |
|---|---|---|---|---|
| Hamilton scale | + | | | |
| Regeneration (Phototrichogram) | | + | + | + |
| Pull-test | | + | + | + |
| Scalp evaluation | | + | + | + |
| Glucose, GTP and differential gene expression tests | | + | | + |
| Adverse events | | | + | + |

### Example 2. Longitudinal, randomized, clinical pilot study for evaluating the healthy properties and tolerability of two treatments on preventing hair loss in a population with non-cicatricial alopecia.

A summary of the results is shown in Table 2.

**Table 2**

| Variables | Treatment 1 | Treatment 2 |
|---|---|---|
| Age | Mean = 49 | Mean = 44.5 |
| Hamilton scale | 50% (5) - III | 40% (4) - II |
| | | 30% (3) - IV |
| Pull test | There are statistically significant differences between the baseline visit (VB) and the 30-day visit (V30), and between the baseline visit and the final 60-day visit (V60). | There are no differences |

| PHOTOTRICHOGRAM | Treatment 1 | Treatment 2 |
|---|---|---|
| Hair density | There are statistically significant differences between each pair of visits, i.e., VB vs. V30, VB vs. V60 and V30 vs. V60. | |
| Telogen | There are statistically significant differences between each pair of visits, i.e., VB vs. V30, VB vs. V60 and V30 vs. V60. | |
| Anagen | There are statistically significant differences between VB vs. V30 and VB vs. V60 | There are statistically significant differences between each pair of visits, i.e., VB vs. V30, VB vs. V60 and V30 vs. V60. |
| % of fine hair | There are statistically significant differences between each pair of visits, i.e., VB vs. V30, VB vs. V60 and V30 vs. V60. | |
| Growth rate | There are statistically significant differences between each pair of visits, i.e., VB vs. V30, VB vs. V60 and V30 vs. V60. | There are statistically significant differences only between V30 vs. V60. |

Discussion of the results:
1. 20 men, with a mean age of 46.75 years who were randomized 1:1 into the two treatment groups, participated in the present study. The participants in the Treatment 1 and 2 groups had a mean age of 49 and 44.5 years, respectively.
2. No statistically significant differences in the Hamilton scale were detected between the participants assigned to each treatment group.

With respect to the Pull test:
3. There were statistically significant differences in the Treatment 1 group among the Pull test means in the 3 visits (p=0 < 0.05), and the differences were found in the comparisons between the day 0 visit and day 30 visit (p=0.0004), day 0 visit and day 60 visit (p=0.0002). No differences were found between the day 30 and day 60 visits (p=0.1147).
4. There were no statistically significant differences in Treatment 2 group among the Pull test means in the 3 visits (p=0.122).
5. Therefore, there is a statistically significant difference in favor of Treatment 1 in the Pull test results.

With respect to Phototrichogram - Treatment 1:
6. There were statistically significant differences (p=0 < 0.05) in the variable hair density means. The differences were detected in all the visits made: day 0 visit and day 30 visit (p=0.000), day 0 visit and day 60 visit (p=0.000) and day 30 visit and day 60 visit (p=0.000).
7. There were statistically significant differences (p=0 < 0.05) in the means of the number of hairs in the telogen phase. The differences were detected in all the visits made: day 0 visit and day 30 visit (p=0.001), day 0 visit and day 60 visit (p=0.001) and day 30 visit and day 60 visit (p=0.001).
8. There were statistically significant differences (p=0 < 0.05) in the means of the number of hairs in the anagen phase. The differences were detected specifically in the day 0 visit and day 30 visit (p=0.000), day 0 visit and day 60 visit (p=0.000).
9. There were statistically significant differences in the ratio of the number of hairs in the anagen/telogen phase in the 3 visits (p=0) and comparing the baseline visit with visit 2 (p=0.000), baseline visit with final visit (p=0.002) and day 30 visit 2 with final visit (p=0.006).
10. Statistically significant differences (p=0 < 0.05) were found in the means of the variable percentage of fine hair (diameter less than 40 µm). The differences were detected in all the visits made: day 0 visit and day 30 visit (p=0.006), day 0 visit and day 60 visit (p=0.005), day 30 visit and day 60 visit (p=0.003).
11. Statistically significant differences (p=0 < 0.05) were found in the means of the variable growth rate. The differences were detected in all the visits made: day 0 visit and day 30 visit (p=0.000), day 0 visit and day 60 visit (p=0.000), day 30 visit and day 60 visit (p=0.000).

With respect to Phototrichogram - Treatment 2:
12. There were statistically significant differences (p=0 < 0.05) in the variable hair density means. The differences were detected in all the visits made: day 0 visit and day 30 visit (p=0.010), day 0 visit and day 60 visit (p=0.000) and day 30 visit and day 60 visit (p=0.003).
13. There were statistically significant differences (p=0 < 0.05) in the means of the variable number of hairs in the telogen phase. The differences were detected in all the visits made: day 0 visit and day 30 visit (p=0.000), day 0 visit and day 60 visit (p=0.001) and day 30 visit and day 60 visit (p=0.008).
14. There were statistically significant differences (p=0 < 0.05) in the means of the variable number of hairs in the anagen phase. The differences were detected in all the visits: day 0 visit and day 30 visit (p=0.000), day 0 visit and day 60 visit (p=0.000), day 30 visit and day 60 visit (p=0.001).
15. There were statistically significant differences in the ratio of the number of hairs in the anagen/telogen phase in the 3 visits (p=0) and comparing the baseline visit with visit 2 (p=0.000), baseline visit with final visit (p=0.004) and day 30 visit 2 with final visit (p=0.006).
16. Statistically significant differences (p=0 < 0.05) were found in the means of the variable percentage of fine hair (diameter less than 40 µm). The differences were detected in all the visits made: day 0 visit and day 30 visit (p=0.007), day 0 visit and day 60 visit (p=0.001), day 30 visit and day 60 visit (p=0.004).
17. Statistically significant differences (p=0 < 0.05) were found in the means of the variable growth rate. The differences were detected only in the day 30 visit and day 60 visit (p=0.001).

Comparing the phototrichogram results for both treatments:
18. It is observed that there were statistically significant differences (p<0.05) in the results obtained between the day 0 visit and day 30 visit in favor of Treatment 1 in the following variables: hair density (p=0.000), percentage of fine hair (diameter less than 40 µm) (P=0.005) and growth rate (p=0.001).
19. In the same manner, it is observed that there were statistically significant differences (p<0.05) in the results obtained between the day 0 visit and day 60 visit in favor of Treatment 1 in the following variables: hair density (p=0.000), number of hairs in the anagen phase (p=0.045), percentage of fine hair (diameter less than 40 µm) (P=0.008) and growth rate (p=0.000).
20. There were no statistically significant differences between the results of the ratio of the number of hairs in the anagen/telogen phase, but there was a trend in favor of the Treatment 1 group.

### Example 3. Longitudinal, randomized, clinical pilot study for evaluating the healthy properties and tolerability of two treatments on preventing hair loss in a population with non-cicatricial alopecia.

Results of the non-parametric test for the averages of the telogen and anagen phases for subjects who received hair treatment by means of food supplement or hair lotion.

### For Treatment 1:

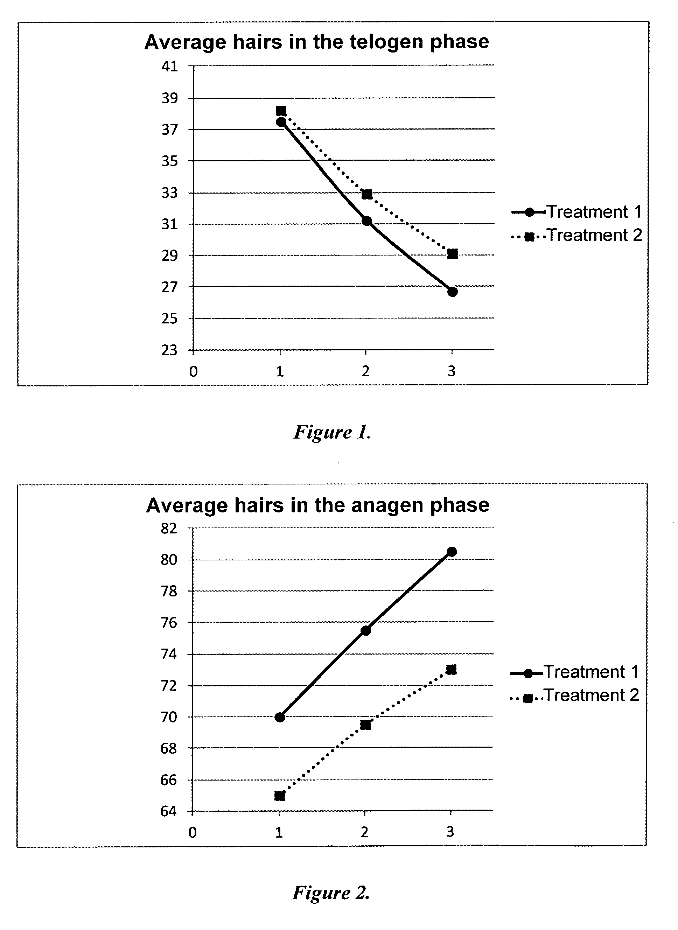

It is confirmed that there are statistically significant differences in the mean amount of hairs in the telogen phase in the subjects who received Treatment 1 in the different visits (p = 0 < 0.05). As observed in Figure 1, there is a statistically significant decrease in the number of hairs in the telogen phase throughout the experimental period (in the 3 visits) in the subjects who received Treatment 1.

Statistically significant differences for a significance level of 5% in the mean amount of hairs in the anagen phase for the subjects who were given the Treatment 1 in the different visits (p = 0 < 0.05) have also been detected. As observed in Figure 2, there is a statistically significant increase in the number of hairs in the anagen phase throughout the experimental period (in the 3 visits) in the subjects who received Treatment 1.

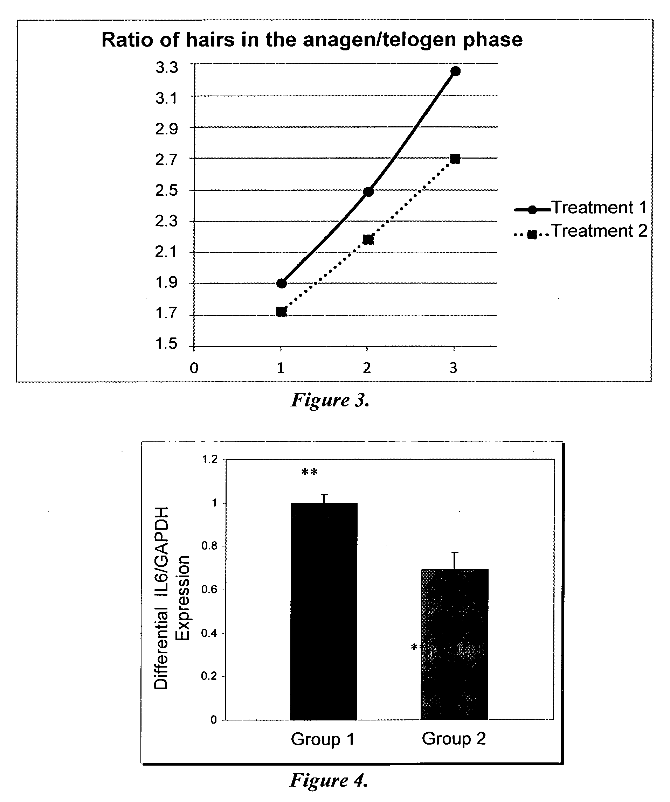

Statistically significant differences in the ratio of hairs in the anagen/telogen phase of Treatment 1 group in the 3 visits of the study (p=0) were also observed. The differences were found in the 3 comparisons between the baseline visit and day 30 visit (p=0.000), in the baseline visit and day 60 visit (p=0.002) and in the day 30 visit and day 60 visit (p=0.006). As can be observed in Figure 3, there were statistically significant differences between the mean values of the variable Ratio of hairs in the anagen/telogen phase of the subjects in the Treatment 1 group for the 3 visits of the study (p<0.05).

### For Treatment 2:

According to the results of the study, the differences of the number of hairs in the telogen phase in the different visits for those subjects who were given Treatment 2 (p = 0 < 0.05) can be confirmed. These differences of mean hairs in the telogen phase were statistically significant between each pair of visits made. As observed in Figure 1, there is a statistically significant decrease in the number of hairs in the telogen phase throughout the experimental period (in the 3 visits) in the subjects who received Treatment 2.

Statistically significant differences for a significance level of 5% (p = 0 < 0.05) in the average number of hairs in the anagen phase in the different visits made in all those subjects who were given Treatment 2 have also been found. As observed in Figure 2, there is a statistically significant increase in the number of hairs in the anagen phase throughout the experimental period (in the 3 visits) in the subjects who received Treatment 2.

Statistically significant differences in the ratio of hairs in the anagen/telogen phase of Treatment 2 group in the 3 visits of the study (p=0) were also observed. The differences were found in the 3 comparisons between the baseline visit and day 30 visit (p=0.000), in the baseline visit and day 60 visit (p=0.004) and in the day 30 visit and day 60 visit (p=0.006). As can be observed in Figure 3, there were statistically significant differences between the mean values of the variable ratio of hairs in the anagen/telogen phase of Treatment 2 group for the 3 visits of the study (p<0.05).

### Example 4. Longitudinal, randomized, clinical pilot study for evaluating the healthy properties and tolerability of two treatments on preventing hair loss in a population with non-cicatricial alopecia.

Results of the analysis and measurement of differential gene expression per treatment group.

The non-parametric test for related samples was applied to detect possible differences between the different visits. Specifically, the Wilcoxon statistic was used to check the equality of averages in the different visits; V1 (0 days of treatment) and V3 (final visit after 60 days of treatment).

### For Treatment 1:

As seen in Table 3, statistically significant differences for a significance level of 5% between visits V1 and V3 for the glucose (p = 0.193 > 0.05) and GPT (p = 1 > 0.05) parameters have not been detected.

**Table 3**

| Non-parametric test for related samples, using the Wilcoxon statistic, in individuals who received Treatment 1 | | | | | | | |
|---|---|---|---|---|---|---|---|
| ANALYSIS: Glucose | n | Mean | SD | Min | Max | Wilcoxon X² | p |
| Selection visit | 10 | 88.7 | 10.4 | 71 | 102 | -1.429 | 0.19 |
| Final visit | 10 | 90.3 | 8.2 | 75 | 99 | | |

| ANALYSIS: GPT | n | Mean | SD | Min | Max | Wilcoxon X² | p |
|---|---|---|---|---|---|---|---|
| Selection visit | 10 | 16 | 3.8 | 10 | 22 | 0 | 1 |
| Final visit | 10 | 16 | 3.8 | 10 | 22 | | |

### For Treatment 2:

As occurred with Treatment 1, statistically significant differences for a significance level of 5% between visits V1 and V3 for the parameters; Glucose (p = 0.102 > 0.05) and GPT (p = 1 > 0.05) have not been detected (see Table 4).

**Table 4**

| Non-parametric test for related samples, using the Wilcoxon statistic, in individuals who received Treatment 2 | | | | | | | |
|---|---|---|---|---|---|---|---|
| ANALYSIS: Glucose | n | Mean | SD | Min | Max | Wilcoxon X² | p |
| Selection visit | 10 | 91.9 | 9.2 | 82 | 111 | -1.704 | 0.10 |
| Final visit | 10 | 94.4 | 5.9 | 86 | 102 | | |

| ANALYSIS: GPT | n | Mean | SD | Min | Max | Wilcoxon X² | p |
|---|---|---|---|---|---|---|---|
| Selection visit | 10 | 17.5 | 3.8 | 13 | 25 | 0 | 1 |
| Final visit | 10 | 17.5 | 3.8 | 13 | 25 | | |

Differential gene expression analysis was performed by means of using microarrays. High density oligonucleotide expression microarrays with Affymetrix GeneChip technology have been used. These microarrays allow measuring the amount of mRNA corresponding to thousands of genes present in the sample (~ 40000 genes) using specific RNA-DNA hybridization technique.

The chips showed the intensity data dependent on the amount of messenger RNA of each gene in Log₂ form. Differential gene expression due to Treatment 1 or 2 with orally or topically administered pomegranate extract, respectively, is calculated using this data by the signal difference between each treatment group after receiving during 60 days administration of pomegranate extract with respect to the control (groups a time zero before receiving the treatments).

Blood samples from participants in the clinical pilot study described in Example 1 were used for the differential gene expression analysis of interleukin-6, KGF, VEGF, TGF-B2, 5-α-reductase type II and aromatase CP-450 genes.

RNA was isolated from lymphocytes isolated from the blood of the participants, before the beginning of treatment (V1) and after the administration of pomegranate extract for 60 days (V3) for gene expression analysis. A pool was made for gene expression analysis by means of microarrays with the RNA from the samples of the same group (V1: time 0 and V3: 60 days).

The following chart shows the differential gene expression results in the case of interleukin-6 after taking pomegranate extract with respect to the initial time. The negative sign indicates inhibition of interleukin-6 gene expression and the value specifies variation with respect to the expression at time 0, which has been normalized to value 1.

| | Treatment 1 (oral administration) | Treatment 2 (topical administration) |
|---|---|---|
| Interleukin-6 | - 2.358 | - 1.618 |

The administration of pomegranate extract by means of treatments 1 (oral) and 2 (topical) causes a statistically significant inhibition of interleukin-6 gene expression in both treatments, though it was greater in the case of the oral administration of the pomegranate extract.

Analysis of the differential gene expression results in the case of the KGF, VEGF, TGF-B2, srd5a 2 (5-α-reductase type II gene) and CYP19A1 (aromatase CP-450 gene) genes did not show statistically significant changes in their expression with respect to the expression at time 0.

### Example 5. Protection against bovine collagen type II-induced cartilage inflammation and degradation provided by pomegranate extract containing pomegranate polyphenols as an active ingredient.

The assay was performed with two groups with 4 mice in each group, group 1 (control) and group 2, that was given pomegranate extract. The mice were fed for 30 days with commercial feed and a daily dose of 12.8 mg of pomegranate extract per mouse in the case of group 2 (equivalent to a daily intake of 400 mg of pomegranate polyphenols/kg body mass equivalent to 160 mg of punicalagins/kg body mass).

After the first 7 days, the mice received an intradermal injection of 100 mg of bovine collagen type II in Freund's complete adjuvant (Difco) at the base of the tail. After inducing arthritis, the mice continued to receive the daily dose of 400 mg of pomegranate polyphenols/kg body mass until completing the 30 days. The mice were weighed weekly and were clinically evaluated five times a week, and leg measurements were recorded three times a week. The mice were sacrificed at the end of the 30 days of study, and the liver, lymph nodes and spleen were extracted from each mouse for RNA isolation and differential gene expression analysis by means of SYBR Green quantitative RT-PCR. Relative quantification was performed using constitutively expressed GAPDH not altered by exogenous factors as the reference gene.

In this animal model, arthritis generally starts in one leg and then spreads to the other legs, which is indicative of the progression of the disease. In the control group that did not receive pomegranate extract, the mean number of legs affected at the end of the study was two. In group two that received oral treatment with pomegranate extract, a tendency to suppress disease progression was shown because in most of the treated animals, the arthritis did not progress affecting two legs.

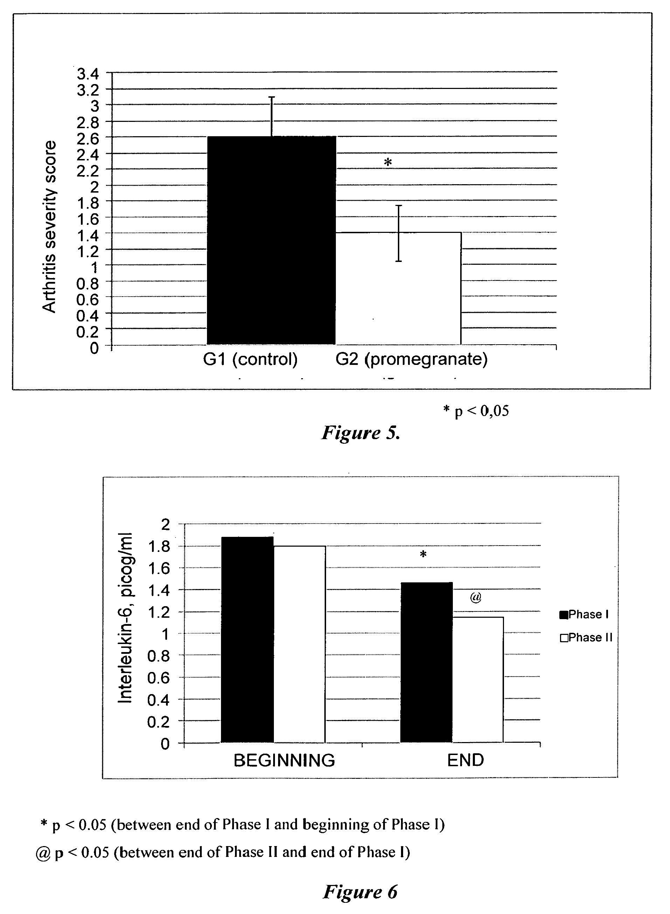

The severity of collagen type II-induced arthritis in mice was evaluated following a scientifically recognized scoring scale. It was found that the average score in the severity of arthritis in group 2 (mice that received oral treatment with pomegranate extract) was significantly less than the average score in the severity of arthritis in group 1 (control mice). The results are shown in Figure 5.

At the end of the 30 days of study, the extremities were removed from the mice and processed for histology. The wrist and metacarpal joints in the front extremities were evaluated, while the ankle and metatarsal joints in the hind legs were evaluated.

Several serial sections of the joints were stained by means of 2 histochemical stains, toluidine blue and aldehyde fuchsia, normally used for staining cartilage matrix components. The histological sections after staining were used to evaluate the loss of matrix components using a scientifically recognized scoring system.

Microphotographs representative of the sections stained with toluidine blue showed that there was a considerable loss of matrix components in the mice in the control group, particularly in the joint surface cartilage and in the regions of inflammatory erosion. The staining shows a high degree of chondrocyte necrosis with empty lacuna in the areas of high inflammatory activity. In contrast, the mice that received the oral administration of 400 mg of pomegranate polyphenols/kg body mass showed a lower loss of matrix component, delaying matrix protein loss in joint surface cartilage. Although the areas with chondrocyte necrosis were still visible, the stain was denser and suggested that the health of the cartilage in general has improved with the oral administration in mice of 400 mg of pomegranate polyphenols/kg body mass.

Taking into consideration the aforementioned data, it can be concluded that the oral administration in mice of 400 mg of pomegranate polyphenols/kg body mass exerted a protective effect on the development of collagen type II-induced arthritis. Differential gene expression results are shown below, the value of the interleukin-6/GAPDH ratio (also see Figure 4) and CD36/GADPH ratio being 1 in the control group (group 1).

| GROUP | CD36/GAPDH Ratio (Normalized) | Interleukin-6/GAPDH Ratio (Normalized) |
|---|---|---|
| 1 | 1 ± 0.05 | 1 ± 0.03 |
| 2 | 0.63 ± 0.07 | 0.69 ± 0.08 |

### Example 6. Nutritional intervention study for quantifying serum interleukin-6 and measuring differential gene expression

The intervention study was performed with 25 participants recruited through the Clinical and Dietetic Nutrition Unit and the Communication Department at Hospital Universitario La Paz in Madrid. In the first phase of the intervention study, the participants took pomegranate extract for 30 days (2 capsules a day of 333 mg of Pomanox^{$} brand pomegranate extract containing 200 mg of pomegranate polyphenols and particularly 100 mg of punicalagins per capsule, which is equivalent to taking 400 mg of pomegranate polyphenols/day). Once the first phase ended, there was an intermediate 15-day washout period during which the participants stopped taking the capsules with pomegranate extract. The second phase of the intervention study was carried out after the washout period in which phase the participants took pomegranate extract for 30 days (4 capsules a day of 333 mg of Pomanox^{$} brand pomegranate extract containing 200 mg of pomegranate polyphenols and particularly 100 mg of punicalagins per capsule, which is equivalent to taking 800 mg of pomegranate polyphenols/day). Fasting blood samples were extracted before taking pomegranate extract and after 30 days of taking two capsules (first phase of the intervention study), and after 15-day washout period and after 30 days of taking four capsules (second phase of the intervention study).

$: standardized in pomegranate polyphenols (60%) and in α + β punicalagins (30%).

A series of criteria which are described below were followed for selecting participants in the intervention study:

### Inclusion criteria

1. Men and women with age comprised between 18 and 75 years.
2. Waist circumference: P 82 (women), P 95 (men)
3. Absence of a family or social environment that prevents complying with dietetic treatment.
4. Suitable level of culture and comprehension of the clinical study.
5. Agreeing to voluntarily participate in the study and giving a written informed consent.

### Exclusion criteria

1. BMI > 30
2. Subjects diagnosed with insulin-dependent diabetes mellitus (type I).
3. Subjects with dyslipidemia undergoing pharmacological treatment.
4. Subjects with arterial hypertension undergoing pharmacological treatment.
5. Smokers or subjects with a high level of alcohol consumption.
6. Subjects who take medicinal products, vitamin, mineral or prebiotic supplements that interfere with the organism's response to the extract studied in the 2 weeks prior to the beginning of the study.
7. Subjects with dementia, mental illness or decreased cognitive function.
8. Subjects with serious diseases (liver, renal, etc.)

Figure 6 shows a graph with the data of interleukin-6 quantification in serum samples from the participants before taking pomegranate extract and after 30 days of taking two capsules (first phase of the intervention study), and after 15-day washout period and after 30 days of taking four capsules (second phase of the intervention study).

The results indicate that there is a significant drop in the interleukin-6 serum levels after taking pomegranate extract for 30 days and that this effect is dose-dependent with the concentration of pomegranate polyphenols administered. A statistically significant drop in interleukin-6 serum level after taking 800 mg of polyphenols/day is observed when compared with the interleukin-6 serum level after taking 2 capsules of pomegranate extract for 30 days, equivalent to 400 mg of polyphenols/day.

Differential gene expression analysis was performed by means of using microarrays. High density oligonucleotide expression microarrays with Affymetrix GeneChip technology (GeneChip® Human Gene 1.0 ST Arrays) have been used. These microarrays allow measuring the amount of mRNA corresponding to thousands of genes present in the sample (~ 40000 genes) using specific RNA-DNA hybridization technique.

The chips showed the intensity data dependent on the amount of messenger RNA of each gene in Log₂ form. Differential gene expression due to 30 day treatment with pomegranate extract is calculated using this data by the signal difference between the participants receiving treatment with respect to those same participants before starting the 30 day treatment.

Fasting blood samples taken before taking pomegranate extract and after taking it for 30 days were used for differential gene expression analysis of the genes.

RNA was isolated from lymphocytes isolated from the blood of the participants before the beginning of treatment and after the administration of pomegranate extract for 30 days for gene expression analysis. A pool was made for gene expression analysis by means of microarrays with the RNA from the samples.

Gene expression analysis was additionally performed with another RNA sample from 6 of the 25 participants by means of individual microarrays for each of the six individuals.

The following chart shows the differential gene expression results in the case of the genes relevant to the present invention. The negative sign indicates inhibition in gene expression and the value specifies variation with respect to expression at time 0 which has been normalized to value 1.

| Gene/Protein | Value of differential gene expression |
|---|---|
| THBS1 gene/Thrombospondin 1 | - 1.882 |
| LPAR6 gene/Lysophosphatidic acid receptor 6 | + 1.673 |

The administration of pomegranate extract for 30 days causes statistically significant inhibition in thrombospondin 1 gene expression. Scientific studies found that thrombospondin 1 plays a critical role in inducing follicle regression and the follicle passing to the catagen phase through its inhibitory effect on angiogenesis. These earlier results identified thrombospondin 1 as a new potential target for therapies directed at hair growth modulation. The present invention demonstrates that the administration of pomegranate extract can be an effective method for the mentioned purpose.

The administration of pomegranate extract for 30 days causes statistically significant activation in LPAR6 gene expression.

In February 2008, researchers at the University of Bonn reported that they had found the genetic base of a different form of hereditary hair loss, opening up a broad path for baldness treatments. They found that the LPAR6 gene causes a rare hereditary form of hair loss called Hypotrichosis simplex. It is the first receptor in humans known to play a role in hair growth. The fact that a receptor plays a specific role in hair growth was unknown until that time by scientists and this new knowledge allowed progressing in the search for therapies for different types of alopecia. Intracellular AMPc is involved in LPAR6 receptor activation. Today, activation of this receptor is known to be important for maintaining hair growth and hair texture.

The present invention demonstrates that the administration of pomegranate extract containing polyphenols as an active ingredient can be an effective method for promoting LPAR6 gene expression and for favoring hair health.

### Example 7. Randomized, longitudinal, intervention study for evaluating the effect of the compositions with pomegranate polyphenols and/or minoxidil on hair growth after topical application.

The population sample consisted of 45 men.

Inclusion/exclusion criteria:
● Male subjects.
● Age between 18 and 65 years old, both included.
● Having non-cicatricial alopecia in stages II to IV according to the Hamilton scale.
● Having not received any treatment for hair loss during the 3 months prior to the beginning of the study.
● Not using hair dyes or treatments susceptible to damaging hair (perms...) within at least two weeks before the beginning of the study.
● Not being allergic to the components of the product under study.
● Suitable level of culture and comprehension of the clinical study.
● Agreeing to voluntarily participate in the study and giving a written informed consent.

The subjects were randomly included in one of the three intervention groups, and treatment was applied according to the sponsor's instructions.

### Experimental treatments:

● Topically administered local treatment by means of hair lotion, consisting of an ethanol and propylene glycol solution in equal parts (hereinafter placebo treatment).
● Topically administered local treatment by means of hair lotion consisting of a 0.2% minoxidil solution (weight/weight) prepared in equal parts of ethanol and propylene glycol (hereinafter Treatment 1).
● Topically administered local treatment by means of hair lotion consisting of a 0.625% pomegranate extract solution weight/weight equivalent to a 0.2% pomegranate polyphenols content weight/weight prepared in equal parts of ethanol and propylene glycol (hereinafter Treatment 2).
● Topically administered local treatment by means of hair lotion consisting of a 0.2% minoxidil solution weight/weight and 0.2% pomegranate polyphenols weight/weight prepared in equal parts of ethanol and propylene glycol (hereinafter Treatment 3).

### The treatments were applied for 30 consecutive days as follows.

The subjects had to apply the treatment daily, at night, in an area of approximately 5 cm in diameter in the center of the front part of the left arm with 30 sprays using the sprayer (approximately 4 ml) of the corresponding hair lotion (T1, T2 or T3), (8 mg of minoxidil in T1, 8 mg of pomegranate polyphenols in T2 and 8 mg of minoxidil and 8 mg of pomegranate polyphenols in T3), while on the other hand 30 sprays using the sprayer (approximately 4 ml) of the placebo treatment were applied in an area of approximately 5 cm in diameter in the center of the front part of the right arm in the same subjects. The lotion was left to act for two hours and was subsequently rinsed, if necessary.

After 30 days of applying the treatments, hair growth was observed in 12 of the subjects in Treatment 3 in the treated area of the left arm, while hair growth was observed in only 4 of the subjects who received Treatment 2 in the treated area of the left arm, and hair growth was not observed in any of the patients in the case of Treatment 1. The placebo treatment did not show hair growth in the treated area of the right arm in any of the patients of the intervention study. In summary, the treatment with the lotion that contained 0.2% minoxidil and 0.2% pomegranate polyphenols clearly showed the synergistic actuation of both active ingredients in hair growth.

It should be highlighted that the minoxidil dose applied in Treatment 3 is lower than that usually applied in treatments with minoxidil.

### Example 8. Randomized, longitudinal, intervention study for evaluating the effect of the compositions with pomegranate polyphenols and/or finasteride on hair growth after oral administration.

The population sample consisted of 30 men.

### Inclusion/exclusion criteria:

● Male subjects.
● Age between 18 and 65 years old, both included.
● Having non-cicatricial alopecia in stages II to IV according to the Hamilton scale.
● Having not received any treatment for hair loss during the 3 months prior to the beginning of the study.
● Not using hair dyes or treatments susceptible to damaging hair (perms...) within at least two weeks before the beginning of the study.
● Not being allergic to the components of the product under study.
● Suitable level of culture and comprehension of the clinical study.
● Agreeing to voluntarily participate in the study and giving a written informed consent.

The subjects were randomly included in one of the three intervention groups, and the corresponding treatment was applied.

### Experimental treatments:

● Pharmacological treatment consisting of 0.3 mg finasteride a day and one capsule a day of Pomanox containing 380 mg pomegranate extract 140 mg of which are pomegranate polyphenols, orally administered (hereinafter Treatment 1).
● Nutricosmetic treatment consisting of one capsule a day of Pomanox containing 380 mg pomegranate extract 140 mg of which are pomegranate polyphenols, orally administered (hereinafter Treatment 2).
● Pharmacological treatment consisting of 0.3 mg finasteride a day orally administered. (hereinafter Treatment 3).

The treatments were applied by the patient for 30 consecutive days, and according to the sponsor's instructions the corresponding composition had to be taken daily after lunch.

The study had 2 phases during development:
C) Selection phase:
   The participants were selected by means of anamnesis and physical examination (selection visit) between four and eight weeks prior to the beginning of the experimental phase.
D) Experimental phase:
   Each subject went through a 30-day experimental period. The subject made 2 visits to the clinic while the experimental phase lasted:
      ● Day 0 (Baseline visit). Evaluation of the capillary area under study by means of phototrichogram and evaluation of the scalp (sebum and/or dandruff). The treatments were supplied.
      ● Day 30 (Visit 2). Evaluation of hair regeneration by means of phototrichogram and
   evaluation of the scalp (sebum and/or dandruff). Recording adverse events.

### Discussion of the results:

1. No statistically significant differences in the Hamilton scale were detected between the participants assigned to each treatment group.

With respect to the evaluation of the scalp (sebum and/or dandruff):
2. No statistically significant differences for a significance level of 5%; p = 1 > 0.05 have been detected in the mean amount of dandruff or in the mean amount of sebum in the different visits made for all those subjects who were given one of the treatments (T1, T2 or T3).

Intergroup statistical analysis (of two in two groups) of the differences found between day 0 visit and day 30 visit was performed with respect to the phototrichogram:

**Table 5. Results of the non-parametric test for k independent samples for evaluating the differences in results of the PHOTOTRICHOGRAM study between participants who received Treatment 1 and 3 between day 0 and day 30 visits.**

| PARAMETER | TREATMENT | N | AVERAGE VALUE |
|---|---|---|---|
| DIF_HAIR DENS | TREATMENT 1 | 10 | 15.10 |
| | TREATMENT 3 | 10 | 5.90 |
| DIF_TELOGEN | TREATMENT 1 | 10 | 10.15 |
| | TREATMENT 3 | 10 | 10.85 |
| DIF_ANAGEN | TREATMENT 1 | 10 | 11.45 |
| | TREATMENT 3 | 10 | 9.55 |
| DIF_FINE_HAIR | TREATMENT 1 | 10 | 6.80 |
| | TREATMENT 3 | 10 | 14.20 |
| DIF_GROWTH_RATE | TREATMENT 1 | 10 | 15.10 |
| | TREATMENT 3 | 10 | 5.90 |

**Table 6. Contrast statistic of the non-parametric test for k independent samples for evaluating the differences in results of the PHOTOTRICHOGRAM study between participants who received Treatment 1 and 3 between day 0 and day 30 visits.**

| Contrast Statistics ^{ab} | | | | | |
|---|---|---|---|---|---|
| | DIF_HAIR_DENS | DIF_TELOGEN | DIF_ANAGEN | DIF_FINE_HAIR | DIF_GROWTH_RATE |
| Chi-square | 13.698 | 0.072 | 1.900 | 7.900 | 12.110 |
| gl | 1 | 1 | 1 | 1 | 1 |
| Asymp. Sig. | 0.000 | 0.768 | 0.168 | 0.005 | 0.001 |

| | | | | | |
|---|---|---|---|---|---|
| a. Kruskal-Wallis Test b. Grouping variable: Treatment | | | | | |

3. As observed in Tables 5 and 6, there are statistically significant differences (p<0.05) between the hair density values (p=0.000), percentage of fine hair (p=0.005) and growth rate (p=0.001) in favor of Treatment 1 when it is compared with Treatment 3 in the results of the differences obtained on the day 30 visit.

**Table 7. Results of the non-parametric test for k independent samples for evaluating the differences in results of the PHOTOTRICHOGRAM study between participants who received Treatment 1 and 2 between day 0 and day 30 visits.**

| PARAMETER | TREATMENT | N | AVERAGE VALUE |
|---|---|---|---|
| DIF_HAIR DENS | TREATMENT 1 | 10 | 15.10 |
| | TREATMENT 2 | 10 | 5.85 |
| DIF_TELOGEN | TREATMENT 1 | 10 | 10.15 |
| | TREATMENT 2 | 10 | 10.85 |
| DIF_ANAGEN | TREATMENT 1 | 10 | 11.45 |
| | TREATMENT 2 | 10 | 8.65 |
| DIF_FINE_HAIR | TREATMENT 1 | 10 | 6.80 |
| | TREATMENT 2 | 10 | 14.00 |
| DIF_GROWTH_RATE | TREATMENT 1 | 10 | 15.10 |
| | TREATMENT 2 | 10 | 5.50 |

**Table 8. Contrast statistic of the non-parametric test for k independent samples for evaluating the differences in results of the PHOTOTRICHOGRAM study between participants who received Treatment 1 and 2 between the day 0 and day 30 visits. Contrast statistics ^{ab}**

| | DIF_HAIR_DENS | DIF_TELOGEN | DIF_ANAGEN | DIF_FINE_HAIR | DIF_GROWTH_RATE |
|---|---|---|---|---|---|
| Chi-square | 12.583 | 0.071 | 4.008 | 7.032 | 14.286 |
| gl | 1 | 1 | 1 | 1 | 1 |
| Asymp. Sig. | 0.000 | 0.790 | 0.045 | 0.008 | 0.000 |

| | | | | | |
|---|---|---|---|---|---|
| a. Kruskal-Wallis Test b. Grouping variable: Treatment | | | | | |

4. As observed in the preceding tables, there are statistically significant differences (p<0.05) between the hair density values (p=0.000), number of hairs in the anagen phase (p=0.045), percentage of fine hair (p=0.008) and growth rate (p=0.000) in favor of Treatment 1 when compared with Treatment 2 in the results of the differences obtained at the day 30 visit.
5. Finally, the same analysis of the contrast statistic of the non-parametric test for k independent samples for evaluating the differences in results of the PHOTOTRICHOGRAM study between the participants who received Treatment 2 and 3 between the day 0 and day 30 visits (data not shown) was performed, being able to conclude that there are no statistically significant differences (p<0.05) between the values of the studied parameters.

In summary, Treatment 1 administered daily for 30 days consisting of taking 0.3 mg of finasteride and 140 mg of pomegranate polyphenols clearly showed the synergistic actuation of both active ingredients in hair growth.

It should be highlighted that the dose of finasteride applied in Treatment 3 is less that that normally applied in treatments with finasteride (1 mg/day).

### Example 9. In vitro assays in a model of human hair follicles to study the effect of interleukin-6 on hair elongation and of DHT on the release of interleukin-6.

Hair follicles isolated from the scalp that were cultured for 7 days in the presence or absence of the dose of interleukin-6 to be tested were used as an *in vitro* model. Specifically, three doses were tested: 0, 25 and 100 nanog/mL. Average hair elongation at the three tested doses was 1.85 mm in the absence of interleukin-6, 1.48 mm and 1.04 mm for the concentrations of 25 and 100 nanog/mL, respectively. In conclusion, interleukin-6 inhibited hair elongation in a dose-dependent manner.

In another independent study, *in vitro* cultures of dermal papilla hair follicle cells grown using DMEM as a culture medium were seeded at a density of 1.5 x 10⁵ cells per 50 mm of plate surface and incubated overnight. Then 3 consecutive washes with PBS were performed and DMEM culture medium supplemented with variables amounts of DHT was added to each of the cultures; specifically three doses were tested: 0, 25 and 100 nanoM. The culture medium was sampled after 24 hours to measure the concentration of interleukin-6 by means of ELISA. The mean concentration of interleukin-6 found in the culture media ELISA analyses was 270, 1270 and 2190 picog/mL for the three doses of DHT (0, 25 and 100 nanoM) tested, clearly showing that DHT caused the release of interleukin-6 due to dermal papilla hair follicle cells. These results strongly suggest that DHT binding to its receptors in dermal papilla hair follicle cells causes the release of interleukin-6 inhibiting hair elongation.

### Example 10. Assay in an animal model to determine bone mineral density

The assay was performed with 40 Sprague Dawley rats (150-180 g) maintained in light/dark conditions in 12 hour cycles at a temperature of 22±2°C and 60% humidity. The rats were fed with a standard diet for 30 days, and two groups were supplemented with pomegranate extract.

The rats were divided into 4 groups:
- Group 1. Rats with simulated operation (control group).
- Group 2. Ovariectomized rats.
- Group 3. Ovariectomized rats that consumed pomegranate extract (dose of 200 mg of pomegranate polyphenols/kg of weight/day equivalent to 80 mg of punicalagins/kg of weight/day).
- Group 4. Ovariectomized rats that consumed pomegranate extract (dose of 400 mg of pomegranate polyphenols/kg of weight/day equivalent to 160 mg of punicalagins/kg of weight/day).

After the assay the right and left femur of all the rats were isolated from the adjacent tissues to analyze bone mineral density therein.

The bone mineral density (BMD) was measured by means of dual-energy X-ray absorptiometry (DXA or DEXA) or bone densitometry. The measurements were taken in a Hologic QDR-4500 A densitometer.

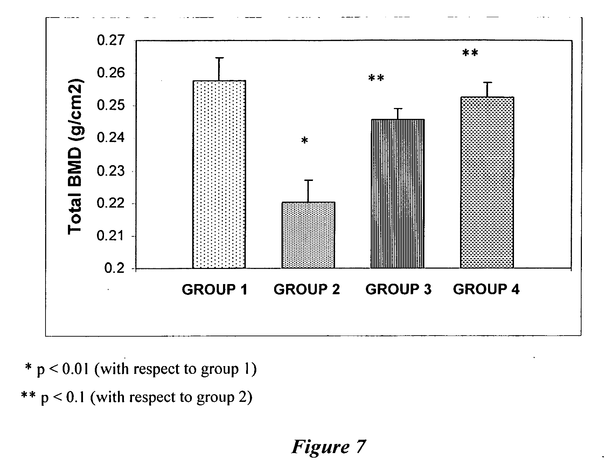

Figure 7 shows the results of total BMD measurements of the femur of rats of each of the four groups.

The results indicate that there are significant differences in total BMD of groups 3 and 4 with respect to group 2, and furthermore that there are no significant differences in total BMD of groups 3 and 4 with respect to group 1 after taking pomegranate extract rich in polyphenols and particularly in punicalagins for 30 days.

## Claims

1. A pomegranate extract containing polyphenols as an active ingredient for use in treating, preventing or co-treating diseases or physiopathological conditions associated with excessive interleukin-6 gene expression and/or excessive activation of the physiological mechanisms mediated by interleukin-6 actuation in mammals.

2. The pomegranate extract for use according to claim 1, wherein the daily dose of polyphenols is in the range between 5 and 1500 mg a day, preferably at least 50 mg/day, more preferably at least 100 mg/day (expressed as gallic acid equivalent).

3. The pomegranate extract for use according to claim 2, wherein the water solubility (ASTM E 1148-02) of the polyphenols is at least 1% (w/w), preferably at least 5% (w/w) and more preferably at least 7% (w/w).

4. The pomegranate extract for use according to any of claims 1 to 3, wherein use is for slowing down bone mass loss in mammals.

5. The pomegranate extract for use according to any of claims 1 to 3, wherein use is for slowing down cartilage loss in mammals.

6. The pomegranate extract for use according to any of claims 1 to 3, wherein use is for the treatment of non-cicatricial alopecias, including increasing hair growth, reducing hair loss, increasing hair follicle viability, or increasing hair follicle cell viability in mammals.

7. A composition containing a pomegranate extract, said extract containing polyphenols as an active ingredient, for use in preventing, treating or co-treating diseases or physiopathological conditions associated with excessive interleukin-6 gene expression and/or excessive activation of the physiological mechanisms mediated by interleukin-6 actuation in mammals.

8. The composition for use according to claim 7, wherein the pomegranate extract is according to claim 2 or 3.

9. The composition according to claim 7 or 8, wherein said use is selected from the treatment of bone mass loss in mammals, the treatment of cartilage loss in mammals and the treatment of non-cicatricial alopecia, including treatment for increasing hair growth, reducing hair loss, increasing hair follicle viability, or increasing hair follicle cell viability in mammals

10. The composition according to claim 9 for the treatment of alopecia, said composition containing, in addition to pomegranate extract, at least one nutrient selected from the group consisting of biotin, B2, B6, B12, vitamin A, vitamin C, vitamin D, folic acid, Fe, Cu, Se, I, Zn, L-methionine, L-cysteine, for-amino benzoic acid (PABA), linoleic acid, and pantothenic acid.

11. The composition according to claim 9 containing, in addition to pomegranate extract, finasteride.

12. The composition according to claim 11, wherein the daily dose of finasteride is in the range between 0.1 and 0.5 mg a day.

13. The composition according to claim 9 containing, in addition to pomegranate extract, minoxidil at a concentration of 0.05% to 1% weight/weight and said pomegranate extract being at a pomegranate polyphenol concentration of 0.2% to 3.2% weight/weight.

14. A pharmaceutical composition for the treatment of non-cicatricial alopecias, containing finasteride and a pomegranate extract containing polyphenols, wherein the total daily dose of finasteride is in the range between 0.1 and 0.5 mg of finasteride/day and the total daily dose of polyphenols is in the range between 5 and 1500 mg/day, preferably at least 50 mg, and more preferably at least 100 mg/day (expressed as gallic acid equivalent).

15. The pharmaceutical composition according to claim 14, wherein said alopecia is androgenetic alopecia.
